(19) Europäisches Patentamt
European Patent Office
Office européen des brevets

(11) Publication number : **0 329 252 B1**

# (12) EUROPEAN PATENT SPECIFICATION

(45) Date of publication of patent specification : **24.06.92 Bulletin 92/26**

(51) Int. Cl.$^5$ : **C07C 51/14,** C07C 53/122, C07C 67/38, C07C 69/24

(21) Application number : **89200366.6**

(22) Date of filing : **14.02.89**

## (54) Process for the preparation of carboxylic acids or esters thereof.

The file contains technical information submitted after the application was filed and not included in this specification

(30) Priority : **16.02.88 GB 8803535**

(43) Date of publication of application : **23.08.89 Bulletin 89/34**

(45) Publication of the grant of the patent : **24.06.92 Bulletin 92/26**

(84) Designated Contracting States : **BE DE ES FR GB IT NL SE**

(56) References cited : **EP-A- 0 291 117**

(73) Proprietor : **SHELL INTERNATIONALE RESEARCH MAATSCHAPPIJ B.V.**
**Carel van Bylandtlaan 30**
**NL-2596 HR Den Haag (NL)**

(72) Inventor : **Drent, Eit**
**Badhuisweg 3**
**NL-1031 CM Amsterdam (NL)**
Inventor : **Breed, Antonius Johannes Maria**
**Badhuisweg 3**
**NL-1031 CM Amsterdam (NL)**

EP 0 329 252 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

The invention relates to a process for the preparation of carboxylic acids or of esters thereof.

U.S. patent specification 3,168,553 discloses a process in which olefins are carbonylated in the presence of a complex comprising a trialkylphosphine together with cobalt, ruthenium, rhodium or iridium. However, this process requires the use of high pressures, and its selectivity towards the desired product is often unsatisfactory. For instance, the carbonylation of ethylene in the presence of ethanol and $Co_2(CO)_8$ as the catalyst leads to the formation not only of ethyl propionate, but also of large quantities of by-products, such as diethyl ketone and acetaldehyde.

U.S. patent specification 3,646,116 discloses a process for the production of methyl esters of linear alkanoic acids which comprises contacting ethylene with methanol in the presence of not more than about 10 per cent carbon monoxide at a temperature of from about 175 °C to about 250 °C, a reaction pressure of from 1000 psig to about 5000 psig and a molar ratio of ethylene to methanol of from 0.75 to 1 to 10 to 1, in the presence of a catalyst consisting of ruthenium in complex combination with carbon monoxide and a triarylphosphine according to the formula $(R_3P)_2Ru(CO)_3$ wherein R is an aryl or alkaryl group of from 6 to 9 carbon atoms, thereby carbomethoxylating said ethylene either as ethylene or as ethylene growth product having 4 to 6 carbon atoms with formation of methyl esters of acids having one more carbon atom than said ethylene or ethylene growth product. Also this process requires the use of high pressures and its selectivity towards the desired product is still unsatisfactory as clearly may be derived from the examples.

U.S. patent specification 4,313,893 discloses a process for the preparation of oxygenated organic compounds e.g. esters, by reacting an olefin with carbon monoxide and a compound containing a replaceable hydrogen atom, in the presence of a catalyst comprising cobalt or ruthenium carbonyl and a promoter ligand. This promoter ligand is selected from heterocyclic nitrogen compounds and phosphorus or sulphur oxides.

Again this process requires the use of relatively high pressures and the selectivity towards the desired product is still unsatisfactory with reference to the present economical requirements for such conversions.

It will be appreciated that due to the hereinbefore mentioned disadvantages of these prior art processes on the one hand people skilled in the art were inclined to look for improved alternative catalyst systems containing other metals than ruthenium.

On the other hand an extensive research for a process showing an improved selectivity and using rather mild conditions has continued and there is still a need for such an improved process, which may provide the desired fine chemicals, the demand for which is still increasing.

As result of extensive research and experimentation it has now surprisingly been found that carboxylic acids or esters thereof are formed, at a very high selectivity and at relatively low pressure, by contacting an olefinically unsaturated compound with carbon monoxide in the presence of water or an alcohol, and in the presence of a ruthenium compound and a group of acids to be specified hereinafter.

The invention provides a process for the preparation of carboxylic acids or of esters thereof, which comprises contacting an olefinically unsaturated compound with carbon monoxide in the presence of water or an alcohol respectively, and a catalytic system, characterized in that the catalytic system may be obtained by combining:

component (a) - a ruthenium compound, and
component (b) - an acid compound according to the general formula

$${}_{m}(O=)X-(R)_{n} \qquad (I)$$

wherein X represents phosphorus, antimony, arsenic, molybdenum or tungsten and preferably phosphorus, molybdenum or tungsten, wherein m = 1 or 2, wherein n = 1, 2 or 3 and wherein each R being different or the same, represents a group OM, wherein M represents a hydrogen, metal, ammonium or inorganic cation (such as uranyl), and/or wherein at most two of the groups R independently represent an alkyl, aryl or aralkyl moiety (preferably methyl, ethyl, n-propyl, isopropyl, n-butyl, isobutyl, tert.butyl, phenyl, naphthyl, benzyl, phenylethyl, optionally substituted by one or more halogen atoms or alkyl groups of 1-4 carbon atoms (more preferably methyl or ethyl)), and/or wherein at most one of the groups R represents a moiety of the formula

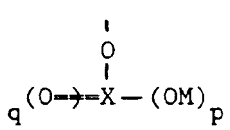

(II)

(wherein p = 1 or 2, and q = 0, 1 or 2), with the proviso that (2m + n) and (2q + p + 1) are equal to the valency of X. The valency of X typically is +3 or +5, preferably +5.

Preferably as component (b) is used a member selected from the group consisting of hydrophosphoric acid, pyrophosphoric acid, hypophosphoric acid, orthophosphoric acid, phenylphosphonic acid, metatungstic acid, orthotungstic acid, phosphomolybdic acid, phosphotungstic acid, molybdic phosphoric acid, molybdic arsenic acid and mixtures thereof and their salts.

The selectivity to a certain compound, as used throughout this specification, expressed in a percentage, is defined herein as 100 x c:d, wherein "c" is the amount of starting olefinically unsaturated compound that has been converted into the desired compound and "d" is the total amount of starting olefinically unsaturated compound that has been converted.

It is a feature of the present invention that the catalytic system is very stable and can be used for a relatively long time therefore; e.g. no plating out of metallic ruthenium has been observed in contrast with similar palladium compound containing catalysts.

The component (b) may be a salt or an acid or a mixture thereof. The salts used as component (b) may have a great variety of cations. Very good results have been obtained with cations of transition metals, particularly zinc or copper, alkali metals and particularly sodium, potassium or lithium, alkaline earth metals and ammonium ions. Good results have also been obtained with uranyl ($UO_2^{2+}$) and related salts.

It will be appreciated that salts may be optionally mixed with crown ethers, e.g. 18-crown-6 or 12-crown-4, to improve their solubility in organic media.

Components (a) and (b) may be the same, i.e. when the ruthenium compound has an anion derived from a compound according to formula I.

Examples of suitable ruthenium compounds are ruthenium oxides and ruthenium salts. Preferably ruthenium trisacetylacetonate, or ruthenium carbonyl complex is used.

The quantity of component (a) used may vary within wide ranges and is generally in the range between $10^{-6}$ and $10^{-1}$ and preferably between $10^{-5}$ and $10^{-2}$ gram-atom ruthenium per mol starting olefinically unsaturated compound.

The quantity of component (b) may also vary within wide ranges; component (b) is preferably present in a quantity in the range of from 0.1 to 100 and particularly from 0.5 to 20 equivalents per gram-atom of ruthenium.

The process according to the present invention may be carried out at a temperature and a pressure which are not critical and may vary within wide ranges. The process is preferably carried out at a temperature in the range of from 100 °C to 250 °C and a pressure in the range of from 5 to 100 bar.

The olefinically unsaturated compound may be an unsubstituted or a substituted alkene or cycloalkene preferably having 2-30, and in particular 2-20, carbon atoms and preferably 1-3 double bonds. The alkene or cycloalkene may be substituted, for instance, with one or more halogen atoms or cyano, ester, alkoxy, hydroxy, carboxy or aryl groups. If the substituents are not inert under the reaction conditions, the carboxylation reaction may be accompanied with other reactions. For instance, the carboxylation of allyl alcohol is accompanied by the esterification of the hydroxy group. Examples of suitable olefinic compounds are ethene, propene, 1-butene, 2-butene, isobutene, the isomeric pentenes, hexenes, octenes and dodecenes, cyclooctadiene-1,5, cyclododecene, cyclododecatriene-1,5,9, allyl alcohol, methyl acrylate, ethyl acrylate, methyl methacrylate, acrylonitrile, acrylamide, N,N-dimethylacrylamide, vinyl chloride, allyl chloride, acrolein, oleic acid, methyl allyl ether and styrene.

The alcohols used in the process according to the invention may be aliphatic, cycloaliphatic or aromatic and may be substituted with one or more substituents, such as mentioned hereinbefore in connection with the olefinically unsaturated compounds to be used as starting material. The alcohol may therefore also be a phenol. The alcohols preferably contain not more than 20 carbon atoms. Examples of suitable alcohols are methanol, ethanol, propanol, isobutanol, tert.butyl alcohol, stearyl alcohol, benzyl alcohol, cyclohexanol, allyl alcohol, chlorocapryl alcohol, ethylene glycol, 1,2-propanediol, 1,4-butanediol, glycerol, polyethylene glycol, 1,6-hexanediol, phenol and cresol.

Special preference is given to alkanols having in the range of from 1 to 10 carbon atoms per molecule. If the alcohol has more than one hydroxy group, different products may be formed, depending on the molar ratios existing between the reagents. For instance, depending on the quantity of olefinically unsaturated compound used, either a mono-ester or a di-ester may be produced from glycerol. The process may be carried out in the simultaneous presence of water and an alcohol, in which case a carboxylic acid and an ester are simultaneously formed. The process according to the present invention is particularly suitable for the preparation of the important solvent methyl propionate, starting from ethylene, carbon monoxide and methanol.

In the process according to the invention the carbon monoxide may be used pure or diluted with an inert gas, such as nitrogen, noble gases or carbon dioxide. Generally the presence of more than 10 %v of hydrogen is undesirable. Generally preference is given to the use of carbon monoxide or a carbon monoxide-containing

gas which contains less than 5 %v of hydrogen.

The molar ratio of the olefinically unsaturated compound to water or alcohol is not critical. The molar ratio between the olefinically unsaturated compound to carbonmonoxide can vary between wide limits but is preferably in the range of from 1:5 to 5:1. When using a mono-olefin and either water or a monohydric alcohol preference is usually given to the use of an excess of the hydroxy compound mentioned. However, when using a polyhydric alcohol to prepare a polyester, it will generally be necessary to use an excess of olefinic compound.

The process according to the invention may be carried out batchwise, continuously or semi-continuously. Generally there is no need for the use of a solvent since usually there will be an excess of one of the reactants - for instance the alcohol - which may serve as a solvent as well. If required, however, a solvent may be used, for instance dimethyl sulphoxide, diisopropyl sulphone, tetrahydrothiophene 1,1-dioxide (also referred to as sulfolane), acetone, chloroform, methyl isobutyl ketone, diglyme (dimethyl ether of di-ethylene glycol) or diisopropyl ether. The primary reaction product of the carbonylation reaction may also be used as a solvent.

The reaction mixtures obtained may be subjected to suitable catalyst and product separating means comprising one or more such steps, for example, as stratification, solvent extraction, distillation, fractionation or adsorption. The catalytic system as well as unconverted starting compounds or solvent, if any, may be recycled, in part or entirely, to the reaction zone.

It will be appreciated that, although the use of catalyst systems composed of a ruthenium compound and phosphorus containing acids like orthophosphoric acid or pyrophosphoric acid was known from e.g. published Japanese Patent Application 61.218539 and U.S. Patent Specification No. 4,599,476, the attractive characteristics of the process of the present invention could certainly not be expected by people skilled in the art, as the type of reaction involved in the present process is totally different from those of respective disclosed processes, i.e. a syngas reaction to manufacture ethanol and a dimerization of olefins respectively.

The following Examples further illustrate the invention however without restricting its scope to these specific embodiments.

The experiments described hereinafter were carried out in a 300 ml magnetically stirred stainless steel autoclave.

The reaction mixtures obtained were analyzed by means of gas-liquid chromatography.

EXAMPLES 1-10

The autoclave was charged with methanol (50 ml, 1.23 mol), ruthenium(acetylacetonate)$_3$ (0.2 mmol) and a compound to be used as "component b", flushed with carbon monoxide, sealed, charged with carbon monoxide until a partial pressure thereof of 20 bar was obtained, charged with ethylene until a partial pressure thereof of 20 bar was obtained, and heated. After a reaction time of 5 hrs at 175 °C the autoclave was allowed to adopt ambient temperature and opened for analysis of the reaction mixture.

Table 1 hereinafter shows for each of the Examples which acid or salt was used as "component (b)", in which amount this compound was used and the reaction rates and the selectivities to methyl propionate.

Table 1

| Example | Component (b) | mmol | Conversion rate mol $C_2H_4$ per gram-atom Ru per h | Selectivity in % to methyl propionate |
|---|---|---|---|---|
| 1 | $(NH_4)_2HPO_4$ | 2 | 90 | 98 |
| 2 | $H_3PO_4$+LiOH | 2+2 | 180 | 95 |
| 3 | $H_3PO_4$+KOH | 2+2 | 165 | 96 |
| 4 | $H_3PO_4$ | 2 | 160 | 95 |
| 5 | $H_2PO_3C_6H_5$ | 2 | 160 | 95 |
| 6 | $H_2WO_4$ | 2 | 70 | 92 |
| 7 | $HPO_3$ | 2 | 90 | 96 |
| 8 | $H_4P_2O_7$ | | 180 | 96 |
| 9 | $H_2MoO_4$ | | 20 | 95 |
| 10 | $H_3PO_4$+(butyl)$_4$NOH | 2+2 | 180 | 95 |
| Comparative Example | none | | only traces of methyl propionate | - |

EXAMPLE 11

An experiment was carried out in about the same way as described for Examples 1-10, with the difference that 0.07 mmol $Ru_3(CO)_{12}$ was used instead of Ru(acetylacetonate)$_3$, while as component (b) 2 mmol $H_3PO_4$ was used.

The average conversion rate was found to be 170 mol ethylene per gram-atom ruthenium per hour and the selectivity to methyl propionate was found to be 96%.

EXAMPLE 12

An experiment was carried out in about the same way as described for Examples 1-10, with the difference that as component (b) 0.33 mol $H_3[P(Mo_3O_{10})_4]$ or phosphomolybdic acid was used.

As average conversion rate was found 80 mol ethylene per gram-atom ruthenium per hour and as selectivity to methyl propionate 96%.

EXAMPLE 13

An experiment was carried out in about the same way as described for Examples 1-10, with the difference that instead of ethylene 20 ml $\alpha$-octene was used, whereas the initial partial pressure of carbon monoxide was 10 bar.

As component (b) 2 mmol $H_3PO_4$ was used. An average conversion rate of 45 mol of $\alpha$-octene/gat Ru.hr was found, whereas the selectivity to methyl nonanoates was 96% and the linearity of the obtained methyl nonanoates was 66%.

EXAMPLE 14

An experiment was carried out in about the same way as described for Examples 1-10, with the difference that 45 ml of diglyme and 15 ml of water were added to the autoclave instead of 50 ml of methanol. As component (b) 2 mmol of $H_3PO_4$ were used. The reaction time was 2.5 hrs.

As conversion rate of ethylene was found 230 mol per gram-atom ruthenium per hour and as selectivity to propionic acid was found 90%.

## Claims

1. Process for the preparation of carboxylic acids or esters thereof by contacting an olefinically unsaturated compound with carbon monoxide in the presence of water or alcohol and in the presence of a catalytic system, characterized in that the catalytic system may be obtained by combining:
component (a) - a ruthenium compound, and
component (b) - an acid compound according to the general formula

$$_{m}(O{=}){=}X-(R)_{n} \qquad (I)$$

wherein X represents phosphorus, antimony, arsenic, molybdenum or tungsten, wherein m - 1 or 2, n - 1, 2 or 3, and each R being different or the same represents a group OM, (wherein M represents a hydrogen, metal, ammonium or inorganic cation), and/or at most two of the groups R independently represent an alkyl, aryl or aralkyl moiety, and/or at most one of the group R represents a moiety of the formula

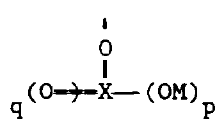

(II)

(wherein p - 1 or 2, and q = 0, 1 or 2), with the proviso that (2m + n) and (2q + p + 1) are equal to the valency of X.

2. A process according to claim 1 characterized in that X represents phosphorus, molybdenum or tungsten.

3. A process according to claim 1 or 2 characterized in that a component (b) is used wherein R represents an OM group or methyl, ethyl, n-propyl, isopropyl, n-butyl, isobutyl, tert.butyl, phenyl, naphthyl, benzyl,

phenylethyl, optionally substituted by one or more halogen atoms or alkyl groups of 1-4 carbon atoms.

4. A process according to any of claims 1-3 characterized in that as component (b) is used a member selected from the group consisting of hydrophosphoric acid, pyrophosphoric acid, hypophosphoric acid, orthophosphoric acid, phenylphosphonic acid, orthotungstic acid, metatungstic acid, phosphomolybdic acid, phosphotungstic acid, molybdic phosphoric acid, molybdic arsenic acid and mixtures thereof and their salts.

5. A process according to any of claims 1-4 characterized in that as component (b) a salt is used selected from zinc, copper, alkali metals, alkaline earth metals and ammonium salts.

6. A process according to any of claims 1-5 characterized in that as component (a) ruthenium trisacetylacetonate or ruthenium carbonyl complex is used.

7. A process according to any of claims 1-6 characterized in that the quantity of component (a) used is in the range between $10^{-6}$ and $10^{-1}$ gram-atom ruthenium per mol starting olefinically unsaturated compound.

8. A process according to any of claims 1-7 characterized in that component (b) is present in a quantity in the range of from 0.1 to 100 equivalents per gram-atom of ruthenium.

9. A process according to any of claims 1-8 characterized in that it is carried out at a temperature in the range of from 100 °C to 250 °C and a pressure in the range of from 5 to 100 bar.

10. A process according to any of claims 1-9 characterized in that as olefinically unsaturated compound an unsubstituted or a substituted alkene or cycloalkene having 2-20 carbon atoms and 1-3 double bonds is used.

11. A process according to any of claims 1-10 characterized in that an alcohol containing not more than 20 carbon atoms is used.

## Patentansprüche

1. Verfahren zur Herstellung von Carbonsäuren oder Estern davon durch Zusammenbringen einer olefinisch ungesättigten Verbindung mit Kohlenmonoxid in Gegenwart von Wasser oder Alkohol und in Gegenwart eines katalytischen Systems, dadurch gekennzeichnet, daß das katalytische System erhalten werden kann durch Zusammenbringen von

Komponente (a) - einer Rutheniumverbindung, und

Komponente (b) - einer sauren Verbindung der allgemeinen Formel

$$_{m}(O\rightarrow)\!-\!X\!-\!(R)_{n} \qquad (I)$$

in der X Phosphor, Antimon, Arsen, Molybdän oder Wolfram bedeutet, wobei m = 1 oder 2, n = 1, 2 oder 3 ist und jedes R, die gleich oder verschieden sein können, eine Gruppe OM bedeutet (wobei M ein Wasserstoff-, Metall- Ammonium- oder anorganisches Kation bedeutet) und/oder höchstens zwei der Reste R unabhängig eine Alkyl-, Aryl- oder Aralkyleinheit bedeuten und/oder höchstens einer der Reste R eine Einheit der Formel

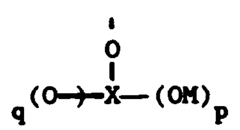

(II)

bedeutet (wobei p = 1 oder 2 und q = 0, 1 oder 2 ist) mit der Maßgabe, daß (2m + n) und (2q + p + 1) gleich sind der Wertigkeit von X.

2. Verfahren nach Anspruch 1, dadurch gekennzeichnet, daß X Phosphor, Molybdän oder Wolfram bedeutet.

3. Verfahren nach Anspruch 1 oder 2, dadurch gekennzeichnet, daß eine Komponente (b) angewandt wird, bei der R eine OM-Gruppe oder Methyl, Ethyl, n-Propyl, Isopropyl, n-Butyl, Isobutyl, tert.Butyl, Phenyl, Naphthyl, Benzyl, Phenylethyl, gegebenenfalls substituiert durch ein oder mehrere Halogenatome oder Alkylgruppen mit 1-4 Kohlenstoffatomen, bedeutet.

4. Verfahren nach einem der Ansprüche 1 bis 3, dadurch gekennzeichnet, daß als Komponente (b) eine Substanz verwendet wird, ausgewählt aus der Gruppe bestehend aus Phosphorsäure, Pyrophosphorsäure, Hypophosphorsäure, Orthophosphorsäure, Phenylphosphonsäure, Orthowolframsäure, Metawolframsäure, Phosphormolybdänsäure, Phosphorwolframsäure, Molybdänphosphorsäure, Molybdänarsensäure und Gemischen daraus und ihren Salzen.

5. Verfahren nach einem der Ansprüche 1 bis 4, dadurch gekennzeichnet, daß als Komponente (b) ein Salz

verwendet wird, ausgewählt aus Zink-, Kupfer-, Alkalimetall-, Erdalkalimetallund Ammoniumsalzen.

6. Verfahren nach einem der Ansprüche 1 bis 5, dadurch gekennzeichnet, daß als Komponente (a) Ruthenium-trisacetylacetonat oder Rutheniumcarbonyl-Komplex verwendet wird.

7. Verfahren nach einem der Ansprüche 1 bis 6, dadurch gekennzeichnet, daß die Menge an verwendeter Komponente (a) im Bereich zwischen $10^{-6}$ und $10^{-1}$ gAtom Ruthenium pro mol olefinisch ungesättigter Aussansverbindung beträgt.

8. Verfahren nach einem der Ansprüche 1 bis 7, dadurch gekennzeichnet, daß die Komponente (b) in einer Menge im Bereich von 0,1 bis 100 Äq pro gAtom Ruthenium vorhanden ist.

9. Verfahren nach einem der Ansprüche 1 bis 8, dadurch gekennzeichnet, daß es bei einer Temperatur im Bereich von 100°C bis 250°C und einem Druck im Bereich von 5 bis 100 bar durchgeführt wird.

10. Verfahren nach einem der Ansprüche 1 bis 9, dadurch gekennzeichnet, daß als olefinisch ungesättigte Verbindung ein unsubstituiertes oder ein substituiertes Alken oder Cycloalken mit 2 bis 20 Kohlenstoffatomen und 1 bis 3 Doppelbindungen verwendet wird.

11. Verfahren nach einem der Ansprüche 1 bis 10, dadurch gekennzeichnet, daß ein Alkohol mit nicht mehr als 20 Kohlenstoffatomen verwendet wird.

## Revendications

1. Procédé pour la préparation d'acides carboxyliques ou de leurs esters par mise en contact d'un composé oléfiniquement insaturé avec de l'oxyde de carbone en présence d'eau ou d'alcool et en présence d'un système catalytique, caractérisé en ce que le système catalytique peut être obtenu en combinant :
un constituant (a) : un composé du ruthénium, et
un constituant (b) : un composé acide de la formule générale

$$_{m}(O{=}X-(R)_{n} \qquad (I)$$

où X représente du phosphore, de l'antimoine, de l'arsenic, du molybdène ou du tungstène, m = 1 ou 2, n = 1, 2 ou 3 et les R, qui peuvent être identiques ou différents, représentent chacun un groupe OM (où M représente un cation d'hydrogène, de métal, d'ammonium ou un cation inorganique) et/ou au maximum deux des groupes R indépendamment représentent une portion alcoyle, aryle ou aralcoyle, et/ou au maximum un des groupes R représente une portion de la formule

$$_{q}(O{=}\overset{\overset{|}{O}}{\underset{}{X}}-(OM)_{p} \qquad (II)$$

(où p = 1 ou 2 et q = 0, 1 ou 2), avec la condition que (2m + n) et (2q + p + 1) sont égaux à la valence de X.

2. Un procédé selon la revendication 1, caractérisé en ce que X représente du phosphore, du molybdène ou du tungstène.

3. Un procédé selon la revendication 1 ou 2, caractérisé en ce qu'on utilise un constituant (b) dans lequel R représente un groupe OM ou un groupe méthyle, éthyle, n-propyle, isopropyle, n-butyle, isobutyle, tert-butyle, phényle, naphtyle, benzyle, phényléthyle, éventuellement substitué par un ou plusieurs atomes d'halogènes ou groupes alcoyle de 1-4 atomes de carbone.

4. Un procédé selon l'une quelconque des revendications 1-3, caractérisé que comme constituant (b) on utilise un membre choisi dans le groupe constitué par l'acide hydrophosphorique, l'acide pyrophosphorique, l'acide hypophosphorique, l'acide orthophosphorique, l'acide phénylphosphorique, l'acide orthotungstique, l'acide métatungstique, l'acide phosphomolybdique, l'acide phosphotungstique, l'acide molybdique-phosphorique, l'acide molybdique-arsénique et leurs mélanges ainsi que leurs sels.

5. Un procédé selon l'une quelconque des revendications 1-4, caractérisé en ce comme constituant (b) on utilise un sel choisi parmi des sels de zinc, de cuivre, de métaux alcalins, de métaux alcalino-terreux et d'ammonium.

6. Un procédé selon l'une quelconque des revendications 1-5, caractérisé en ce que comme constituant (a) on utilise du trisacétylacétonate de ruthénium ou un complexe de ruthénium-carbonyle.

7. Un procédé selon l'une quelconque des revendications 1-6, caractérisé en ce que la quantité de constituant (a) utilisée est comprise entre $10^{-6}$ et $10^{-1}$ atome-gramme de ruthénium par mole de composé oléfiniquement insaturé de départ.

8. Un procédé selon l'une quelconque des revendications 1-7, caractérisé en ce que le constituant (b) est présent à raison d'une quantité comprise entre 0,1 et 100 équivalents par atome-gramme de ruthénium.

9. Un procédé selon l'une quelconque des revendications 1-8, caractérisé en ce qu'il est mis en oeuvre à une température comprise entre 100°C et 250°C et une pression comprise entre 5 et 100 bars.

10. Un procédé selon l'une quelconque des revendications 1-9, caractérisé en ce que comme composé oléfiniquement insaturé on utilise un alcène ou cycloalcène non-substitué ou substitué ayant 2-20 atomes de carbone et 1-3 doubles liaisons.

11. Un procédé selon l'une quelconque des revendications 1-10, caractérisé en ce qu'on utilise un alcool ne contenant pas plus de 20 atomes de carbone.